# FASCICULE DE BREVET EUROPEEN

(11) **EP 2 854 674 B1**
(45) Date de publication et mention de la délivrance du brevet: **15.11.2017**
(21) Numéro de dépôt: 13731368.0
(22) Date de dépôt: 28.05.2013
(51) Int. Cl.: A61B 17/70, A61B 17/02

(54) **SYSTEME D'INSTRUMENTATION POUR LA REALISATION D'UNE INTERVENTION CHIRURGICALE SUR DES VERTEBRES COMPRENANT DES MOYENS DE BLOCAGE TEMPORAIRE**
INSTRUMENTENSYSTEM ZUR DURCHFÜHRUNG EINES CHIRURGISCHEN EINGRIFFS AN WIRBELN MIT EINER TEMPORÄREN IMMOBILISIERUNGMITTELN
INSTRUMENT SYSTEM FOR CARRYING OUT A SURGICAL PROCEDURE ON THE VERTEBRAE COMPRISING TEMPORARY IMMOBILIZATION MEANS

(30) Priorité: 28.05.2012 FR 1254896
(43) Date de publication de la demande: 08.04.2015
(73) Titulaire: Safe Orthopaedics, 95610 Eragny-sur-Oise (FR)
(72) Inventeur: DROULOUT, Thomas, F-78300 Poissy (FR)
(74) Mandataire: IP Trust
(86) Numéro de dépôt international: PCT/FR2013/051183
(87) Numéro de publication internationale: WO 2013/178933

(56) Documents cités:
- US-A1- 2006 111 712
- US-A1- 2008 262 318
- US-A1- 2009 149 887

## Description

### Domaine de l'invention

La présente invention concerne le domaine des instruments chirurgicaux pour les interventions de stabilisation rachidienne par élément d'ancrage osseux de type vis par voies postérieure ou postéro-latérale.

L'invention concerne plus particulièrement un kit d'instrumentation selon l'invention destiné notamment, mais non exclusivement, à la chirurgie d'ostéosynthèse rachidienne lombaire, thoracique, ou encore cervicale postérieure par voies chirurgicale minimalement invasive ou ouverte.

Lors de dysfonctionnements anatomiques de la colonne vertébrale, on procède à la mise en place d'ancrages osseux de type vis pédiculaire ou vertébrales dans les vertèbres reliées entre elles par des éléments de liaison de type tiges ou plaques.

### Etat de la technique

On connaît de l'état de la technique des systèmes d'instrumentation pour la réalisation d'interventions chirurgicales sur des vertèbres constitué par au moins un tube et au moins une vis rachidienne apte à coopérer avec l'extrémité proximale du tube et présentant une tête à mobilité multiaxiale. On peut citer à titre d'exemple les demandes de brevet US2009/149887 et US2006/111712.

Il peut être également cité la demande WO2011/080426 de la demanderesse. Ce document divulgue une instrumentation pour la fixation d'au moins deux vertèbres rachidiennes par implants d'ancrage osseux de type vis pédiculaires comprenant un élément d'ancrage osseux destinés à être fixé sur une vertèbre, pré-monté sur un tube de montage à usage unique, et un emballage scellé de conditionnement stérile.

Ce document de l'art antérieur concerne également un kit d'instruments pour la pose ou la dépose d'un implant rachidien comportant au moins deux éléments d'ancrage osseux filetés, un organe de liaison de type tige ou plaque reliant mécaniquement les éléments d'ancrage osseux et des éléments de blocage pour verrouiller en position l'élément de liaison par rapport aux éléments d'ancrage, pour réaliser la totalité des gestes chirurgicaux liés à la pose ou la dépose dudit implant, caractérisé en ce que la totalité de ces instruments nécessaires sont à usage unique, conditionnés stérile dans un ou plusieurs emballages scellés.

On connaît également le brevet français FR2874496 décrivant un rétracteur de tissus d'un patient, du type comprenant deux lames présentant respectivement une extrémité proximale et une extrémité distale, lesdites lames étant disposées pour former un canal opératoire ouvert aux extrémités proximales et aux extrémités distales desdites lames, caractérisé en ce que le rétracteur comporte au moins une lame complémentaire pour former un rétracteur à trois lames au moins, lesdites lames s'écartant les unes des autres par pivotement de leurs extrémités distales de sorte à former un canal opératoire élargi de forme conique.

On connaît aussi la demande de brevet européen EP0455282 décrivant un écarteur autostatique comprenant un châssis d'enrouleur polygonal associé à une pluralité de dilatateurs.

Lorsqu'il est inséré profondément dans le corps du patient, il maintient les bords de l'incision ouverts. Les côtés sont reliés de manière articulée les uns aux autres et deux charnières opposées.

Un autre exemple d'écarteur est décrit dans le brevet américain US3509873.

On connaît également de la demande US2008/0262318 un système d'écarteur souple permettant une chirurgie mini-invasive de la colonne vertébrale.

### Inconvénients de l'état de la technique

Ces solutions de rétracteurs de l'art antérieur présentent deux inconvénients majeurs. En premier lieu, ces solutions se traduisent par deux dispositifs distincts :
- un ou plusieurs tubes permettant de maintenir la vis rachidienne et de guider au cours de l'intervention la tige de liaison qui vient s'insérer dans la tête de la vis, puis d'engager et visser un bouchon verrouillant la tige dans la tête de la vis.
- un deuxième dispositif constitué par plusieurs lames apte à maintenir les tissus aux abords de la zone d'intervention.

Dans les solutions de l'art antérieur, ces dispositifs connus occupent un volume important dans la zone chirurgicale, ce qui oblige soit à élargir l'incision, soit à accepter un champ de vision et de travail étroit. Dans les deux cas, le chirurgien est gêné dans l'exécution de l'intervention et du geste chirurgical.

Le deuxième inconvénient est que le deuxième dispositif doit être positionné avant l'insertion des vis. Une fois qu'il est placé dans la zone d'intervention, il vient limiter l'angulation possible des tubes et rend donc plus difficile la visée rachidienne et l'engagement de la zone proximale du tube sur la vis rachidienne lorsque la vis n'est pas pré-montée sur le tube.

Enfin, dans toutes les solutions de l'art antérieur, le rétracteur (deuxième dispositif) est constitué par un instrument chirurgical complexe, nécessitant une stérilisation minutieuse et difficile, en raison des formes complexes et de la présence de multiples articulations, après chaque utilisation. Ce dispositif supplémentaire complexe occasionne par ailleurs un surcoût difficilement supportable au regard des contraintes d'économie de la santé.

### Solution apportée par l'invention

Afin de remédier aux inconvénients de l'art antérieur, la présente invention propose une solution consistant à compléter le ou les tube(s) rachidien(s) par un accessoire simple au moins permettant d'utiliser le tube non seulement pour sa fonction initiale de pose la vis rachidienne, d'insertion de la tige de liaison et de serrage du bouchon, mais aussi de conférer au(x) tube(s) une fonction annexe de rétractation des tissus.

La présente invention est définie par la revendication indépendante 1 tandis que des modes de réalisations préférés sont exposés dans les revendications dépendantes.

A cet effet, l'invention concerne selon son acception la plus générale un système d'instrumentation pour la réalisation d'une intervention chirurgicale sur des vertèbres par voie postérieure ou postéro-latérale, constitué par au moins un tube et au moins une vis rachidienne apte à coopérer avec l'extrémité proximale dudit tube et présentant une tête à mobilité multiaxiale, caractérisé en ce qu'il comporte en outre une calle de blocage temporaire de l'orientation de la tête de la vis.

On entend par l'expression « extrémité distale », l'extrémité la plus éloignée de l'implant, et par l'expression « extrémité proximale », l'extrémité la plus proche de l'implant.

En permettant de bloquer la tête de vis dans n'importe quelle position angulaire par rapport à la tige filetée de la vis, la calle de blocage permet ainsi de maintenir le tube coopérant avec la vis selon une orientation souhaitée. L'angulation des tubes est donc améliorée au regard des systèmes d'instrumentation de l'art antérieur.

On comprend bien que le blocage de l'orientation de la tête de vis, et donc celle du tube, est temporaire en ce sens que la calle de blocage doit être retirée pour poser une tige de liaison sur la tête de vis. La calle de blocage n'intervient donc que pour bloquer temporairement la multi-axialité de la tête de vis et permettre ainsi de procéder à certaines opérations précédent la pose de la tige de liaison et nécessitant le maintien des tubes selon une orientation donnée.

De préférence, ladite calle présente une extrémité semi-cylindrique, d'une forme complémentaire à celle de l'encoche en « U » de réception d'une tige de liaison, prévue sur la tête de la vis.

Selon une première variante, ladite calle de blocage est constituée par l'extrémité proximale d'un insert coulissant à l'intérieur dudit tube.

Selon une deuxième variante, ladite calle de blocage présente un moyen de liaison avec un insert coulissant à l'intérieur dudit tube.

Avantageusement, la calle de blocage est apte à être introduite par l'intérieur du tube, depuis son extrémité distale

Avantageusement, ledit insert présente au moins une nervure de guidage de section transversale complémentaire à celle d'au moins une gorge de guidage prévue dans la cavité formée dans ledit tube.

Selon un mode de mise en oeuvre particulier, ledit tubes est formé de deux demi-coques aptes à coopérer avec la tête d'une vis rachidienne d'une manière permettant un basculement proximal de l'une au moins desdites demi-coques par rapport à la tête de ladite vis rachidienne. De part cette configuration, la fonction de guidage du premier type de dispositif de l'art antérieur et la fonction de rétraction des tissus du deuxième type de dispositif de l'art antérieur (rétracteur) sont réalisées par un seul et même instrument, à savoir le tube.

Selon une première variante, ladite calle de blocage présente un moyen de fixation réversible sur ledit insert.

Selon une deuxième variante, ledit moyen de fixation réversible est constitué par une zone de clipsage.

Avantageusement, ledit moyen de fixation réversible est constitué par une zone de vissage.

Selon un mode de mise en oeuvre avantageux, ledit insert présente une zone de verrouillage par vissage sur ledit tube. Ledit insert est avantageusement constitué par l'insert de mise en place du bouchon de verrouillage de la tête de la vis.

L'invention concerne également une calle de blocage temporaire de l'orientation de la tête de la vis, apte à être introduite par l'intérieur du tube, depuis son extrémité distale, présentant une extrémité semi-cylindrique d'une forme complémentaire à celle de l'encoche en « U » de réception d'une tige de liaison, prévue sur la tête de la vis.

### Description détaillée d'exemples non limitatifs de réalisation

D'autres objets et avantages de l'invention apparaîtront au cours de la description qui suit, faite en référence aux dessins annexés, dans lesquels :
- la figure 1 représente une vue en perspective de deux tubes lors de mise en place des vis dans une vertèbre

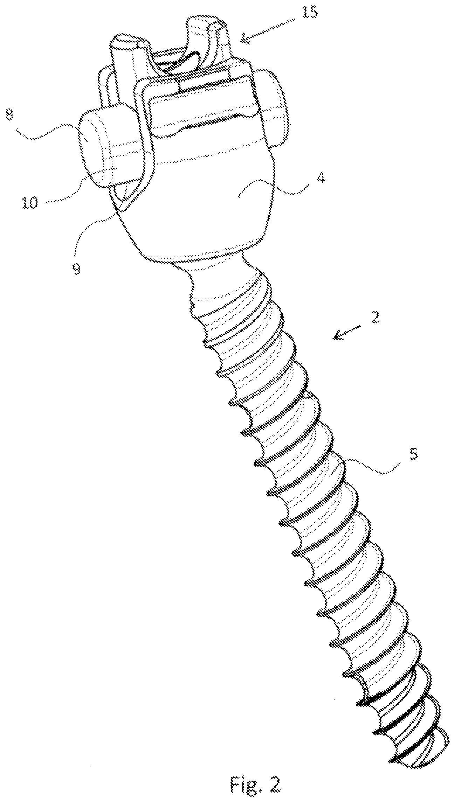

- la figure 2 représente une vue d'une calle selon l'invention associée avec une vis pédiculaire
- la figure 3 représente une vue d'une deuxième variante de réalisation de la calle prolongée par un insert
- la figure 4 représente une vue en section transversale d'un tube avec l'insert et la calle.

### Présentation sommaire des tubes connus dans l'art antérieur.

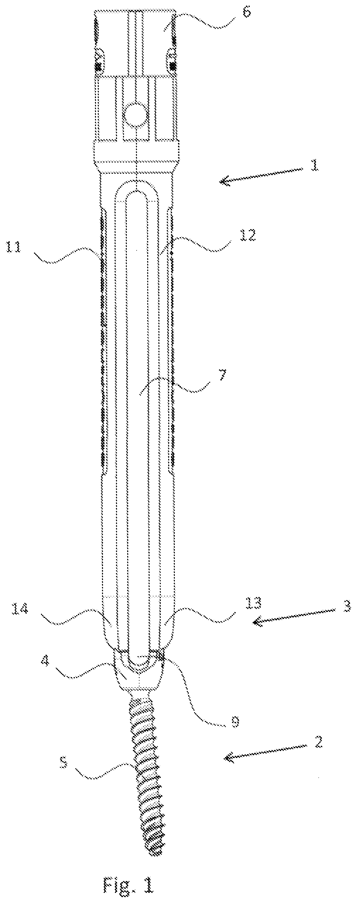

La figure 1 représente une vue de profil d'un tube(1) de fixation rachidienne lors de mise en place d'une vis (2) dans une vertèbre, avec des tubes connus dans l'art antérieur.

Le tube (1) supporte à son extrémité proximale (3) une vis (2) rachidienne (ou « pédiculaire ») qui est avantageusement pré-montée. La liaison entre l'extrémité proximale (3) et la vis (2) est assurée par l'intermédiaire d'une tête (4) présentant une gorge d'accrochage coopérant avec un épaulement prévus à la surface intérieure de l'extrémité proximale (3) du tube (1).

La tête (4) de la vis et la partie filetée (5) coopère par une rotule permettant une orientation multiaxiale, et un blocage par l'intermédiaire de la tige de liaison et d'un bouchon fileté venant appuyer, par l'intermédiaire ou non d'une pièce complémentaire, la tige de liaison contre l'extrémité supérieure de la tige filetée (5) lorsque l'ensemble est en place et correctement orienté.

La vis pédiculaire (2) est destinée à être fixée sur des vertèbres. Elle comprend un moyen d'ancrage osseux (5) prolongé par une tête (4) fendue pour recevoir une tige de liaison intervertébrale non représentée. Lorsque la tige est en place, un bouchon vissable (non représenté) vient se visser dans la tête (4) par un filetage, pour verrouiller l'ensemble.

Le matériau le plus fréquemment utilisé pour la fabrication des vis est le titane. Dans une configuration particulière de l'invention le matériau utilisé pour la fabrication peut être tous matériaux implantables connus ou non à ce jour, comme le Peek, l'inox, le chrome cobalt, ou encore un composite à base de fibre de verre ou de carbone. Des revêtements de type HATCP (HydroxyApatite TriCalcium Phosphate) ou autres peuvent également être appliqués pour améliorer l'ancrage osseux ou bien la tenue mécanique globale de l'implant.

La pose de la vis (2) et la mise en place de la tige, puis son verrouillage avec le bouchon vissable sont assurés par un instrument décrit dans le brevet de l'art antérieur WO2011/080426 dont le contenu est incorporé à la présente demande par référence à cette demande PCT.

Le tube (1) est, dans l'exemple décrit, constitués de deux demi-coques respectivement (11, 12), articulés à leurs extrémités proximales (13, 14) d'une manière autorisant un basculement par rapport à la tête de la vis (4), lorsque les deux demi-coques sont libérées.

Une bague (6) assure le verrouillage des deux demi-coques (11, 12) pour former un tube (1) fermé lors de certaines phases d'utilisation.

Le tube (1) présente des lumières longitudinales (7) permettant l'engagement d'une tige de liaison et son déplacement jusque dans la fente en « U » prévue dans la tête (4) de la vis.

Il est à noter que l'invention n'est pas limitée à la mise en oeuvre de tubes formés de deux demi-coques, et peut s'appliquer également à des tubes constitués d'une seule pièce.

Toutefois, la réalisation sous forme de deux demi-coques assemblées constitue un mode de réalisation préféré.

### Description détaillée d'un exemple de réalisation.

L'invention vise à permettre l'utilisation de tels tubes connus dans l'état de la technique pour assurer accessoirement la rétraction des tissus, sans qu'il ne soit nécessaire d'utiliser un instrument complémentaire tels que les rétracteurs connus dans l'art antérieur.

A cet effet, l'invention comprend une pièce accessoire constituée par une calle (15) venant se loger provisoirement à la place de la tige de liaison, et permettant d'assurer temporairement le blocage de l'articulation de la tête (4) de la vis (2) par rapport à l'axe du filetage (5).

Cette calle (15) vient se loger dans l'espace destiné à la réception d'une tige de liaison et vient en appui sur l'extrémité frontale supérieure de la tige filetée (5), par l'intermédiaire ou non d'une pièce complémentaire, pour assurer son verrouillage par rapport à la tête (4) de la vis.

L'extrémité (8) de la calle (15) présente une épaisseur correspondant à la section de la gorge en « U » (9) destinée à recevoir une tige de liaison, et un bord d'appui frontal(10) semi-cylindrique configuré pour permettre le verrouillage de la partie filetée (5) de la vis par rapport à la tête (4).

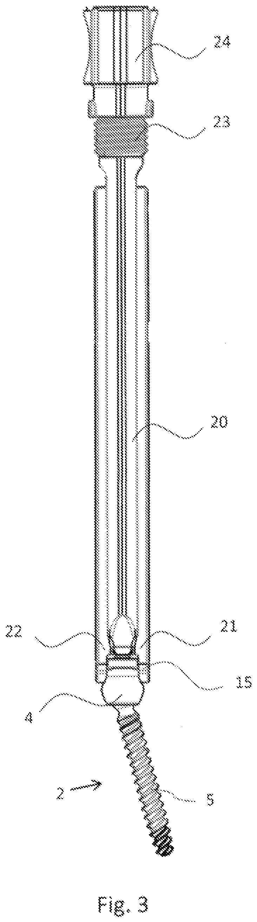

L'extrémité supérieure de la calle (15), opposée à ce bord d'appui (10), présente une zone de fixation complémentaire à l'extrémité proximale d'un insert (20) représenté en figure 3.

Cet insert (20) est avantageusement l'insert connu dans l'art antérieur représenté par la demande WO2011/080426 de la demanderesse, pour la poussée de la tige de liaison et l'introduction du bouchon vissé dans la tête de la vis.

L'extrémité proximale (23) de cet insert (20) est fendue et présente deux pattes (21, 22) délimitant une ouverture habituellement destinée au passage du bouchon vissable.

Le moyen de fixation de la calle (15) présente une forme complémentaire à cette extrémité proximale de l'insert (20) pour permettre une fixation par clipsage. La calle (15) peut ainsi être positionnée sur l'insert, puis introduite grâce à cet insert (20) coulissant par glissement longitudinal dans le tube jusqu'à venir en position dans la tête (4) de la vis (2).

L'insert (20) est ensuite verrouillé par rapport au tube (1) par vissage grâce un filet (23) pivotant prévu à l'extrémité distale de l'insert (20). Lorsque l'insert est vissé sur le tube (1), il exerce une poussée sur la calle (15), qui vient ainsi verrouiller angulairement la tige filetée (5) par rapport à la tête de la vis (4), dans une position dans laquelle les parois du tube (1) écarte les bords de l'incision pratiquée dans les tissus, et faciliter ainsi l'accès à la zone d'intervention chirurgicale.

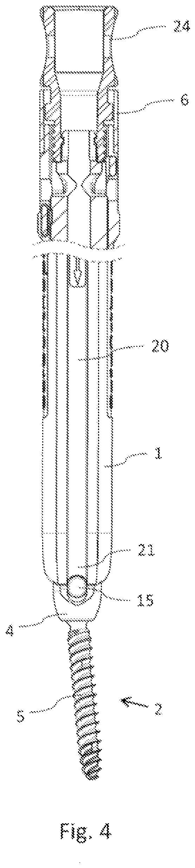

La figure 4 représente une vue en coupe longitudinale du système. L'insert (20) présente à son extrémité distale une tête pivotante (24) munie d'un filetage (23) pouvant coopérer avec un filetage prévu à l'intérieur du tube (1).

Selon une alternative, la calle (15) et l'insert peuvent ne faire qu'une seule pièce.

Selon une autre alternative le moyen de poussée et de verrouillage de l'insert (20) par rapport au tube (1) peut être constitué par une pièce additionnelle.

Le tube (1), l'insert (20) et la calle (15) peuvent être constituées en polymère injecté pour permettre la réalisation d'un système à usage unique.

Selon une autre variante l'insert (20) est configuré pour permettre le pré-chargement du bouchon qui est mis en place dans cavité de réception prévue à l'extrémité proximale de l'insert (20), comme décrit dans la demande WO2011/080426 de la demanderesse. Le bouchon est mis en place au moment de la fabrication du système, et reste en place, notamment pendant l'utilisation du système comme rétracteur de tissus, jusqu'à l'étape de verrouillage de la tige de liaison.

L'utilisation lors d'une intervention chirurgicale de plusieurs systèmes, associé chacun à une vis rachidienne implanté sur des vertèbres consécutives, permet ainsi de distendre les tissus pour former un volume de travail parfaitement accessible, sans qu'il ne soit nécessaire de recourir à des rétracteurs prévus dans l'art antérieur.

## Revendications

1. Système d'instrumentation pour la réalisation d'une intervention chirurgicale sur des vertèbres par voie postérieure ou postéro-latérale, constitué par au moins un tube (1) présentant une extrémité proximale et une extrémité distale, et au moins une vis rachidienne (2) coopérant avec l'extrémité proximale (3) dudit tube (1) et présentant une tête à mobilité multiaxiale, **caractérisé en ce qu'**il comporte en outre un insert (20), apte à coulisser à l'intérieur du tube (1), pourvu en extrémité proximale d'une calle (15) de blocage temporaire de l'orientation de la tête de la vis, ladite calle (15), apte à être introduite par l'intérieur du tube (1) depuis son extrémité distale, présentant une extrémité semi-cylindrique (8) de forme complémentaire à celle de l'encoche en « U » de réception d'une tige de liaison prévue sur la tête de la vis.

2. Système d'instrumentation selon la revendication 1, **caractérisé en ce que** ladite calle (15) de blocage est constituée par l'extrémité proximale de l'insert (20).

3. Système d'instrumentation selon la revendication 1 ou la revendication 2, **caractérisé en ce que** ladite calle (15) de blocage présente un moyen de liaison avec l'insert(20).

4. Système d'instrumentation selon la revendication précédente, **caractérisé en ce que** ledit insert (20) présente au moins une nervure de guidage de section transversale complémentaire à celle d'au moins une gorge de guidage prévue dans la cavité formée dans ledit tube (1).

5. Système d'instrumentation selon la revendication précédente, **caractérisé en ce que** ledit tube est formé de deux demi-coques aptes à coopérer avec la tête (4) d'une vis rachidienne d'une manière permettant un basculement proximal de l'une au moins desdites demi-coques (11, 12) par rapport à la tête de ladite vis rachidienne (2).

6. Système d'instrumentation selon l'une quelconque des revendications 3 à 5, **caractérisé en ce que** ladite calle (15) de blocage présente un moyen de fixation réversible sur ledit insert (20).

7. Système d'instrumentation selon la revendication précédente, **caractérisé en ce que** ledit moyen de fixation réversible est constitué par une zone de clipsage.

8. Système d'instrumentation selon la revendication 6, **caractérisé en ce que** ledit moyen de fixation réversible est constitué par une zone de vissage.

9. Système d'instrumentation selon l'une quelconque des revendications 3 à 8, **caractérisé en ce que** ledit insert présente une zone de verrouillage par vissage sur ledit tube (1).

10. Système d'instrumentation selon la revendication 1 ou la revendication 2, **caractérisé en ce que** la calle (15) de blocage et l'insert (20) font qu'une seule pièce.

## Patentansprüche

1. Instrumentensystem zur Durchführung eines chirurgischen Eingriffs an den Wirbelkörpern durch dorsalen oder dorsolateralen Zugang, bestehend aus mindestens einer Röhre (1), welche ein proximales Ende und ein distales Ende aufweist, und mindestens einer Wirbelsäulenschraube (2), die mit dem proximalen Ende (3) besagter Röhre (1) zusammen wirkt und einen multiaxial beweglichen Kopf aufweist, **dadurch gekennzeichnet, dass** es des Weiteren einen Einsatz (20) umfasst, der im Inneren der Röhre (1) verschoben werden kann, und der am proximalen Ende mit einem Riegelkeil (15) zur vorübergehenden Blockierung der Ausrichtung des Schraubenkopfes versehen ist, wobei besagter Riegelkeil (15), der vom Inneren der Röhre (1) von deren distalen Ende aus eingesetzt werden kann, ein halbzylindrisches Ende (8) aufweist, dessen Form komplementär zur u-förmigen Ausbuchtung zur Aufnahme eines auf dem Schraubenkopf vorgesehenen Verbindungsstabes ist.

2. Instrumentensystem nach Anspruch 1, **dadurch gekennzeichnet, dass** besagter Riegelkeil (15) aus dem proximalen Ende des Einsatzes (20) besteht.

3. Instrumentensystem nach Anspruch 1 oder Anspruch 2, **dadurch gekennzeichnet, dass** besagter Riegelkeil (15) ein Mittel zur Verbindung mit dem Einsatz (20) aufweist.

4. Instrumentensystem nach dem vorhergehenden Anspruch, **dadurch gekennzeichnet, dass** besagter Einsatz (20) mindestens einen Führungssteg mit einer Querschnittsfläche aufweist, die komplementär ist zur Querschnittsfläche von mindestens einer Führungsnut, die in dem durch besagte Röhre (1) geformten Hohlraum vorgesehen ist.

5. Instrumentensysten nach dem vorhergehenden Anspruch, **dadurch gekennzeichnet, dass** besagte Röhre aus zwei Halbschalen geformt wird, die in der Lage sind, mit dem Kopf (4) einer Wirbelsäulenschraube so zusammen zu wirken, dass mindestens eine der besagten Halbschalen (11, 12) relativ zum Kopf der besagten Wirbelsäulenschraube (2) proximal gekippt werden kann.

6. Instrumentensysten nach einem der Ansprüche 3 bis 5, **dadurch gekennzeichnet dass** besagter Riegelkeil (15) ein Mittel zur reversiblen Befestigung auf besagtem Einsatz (20) aufweist.

7. Instrumentensystem nach dem vorhergehenden Anspruch, **dadurch gekennzeichnet, dass** besagtes reversibles Befestigungsmittel aus einem Klemmbereich besteht.

8. Instrumentensystem nach Anspruch 6, **dadurch gekennzeichnet**, das besagtes reversibles Befestigungssystem aus einem Verschraubungsbereich besteht.

9. Instrumentensystem nach einem der Ansprüche 3 bis 8, **dadurch gekennzeichnet, dass** besagter Einsatz einen Verriegelungsbereich durch Verschraubung auf besagter Röhre (1) aufweist.

10. Instrumentensystem nach Anspruch 1 oder Anspruch 2, **dadurch gekennzeichnet, dass** der Riegelkeil (15) und der Einsatz (20) ein einziges Stück bilden.

## Claims

1. An instrument system for carrying out a surgical procedure on vertebrae from a posterior or posterolateral approach, consisting of at least one tube (1) having a proximal end and a distal end, and at least one spinal screw (2) capable of engaging with the proximal end (3) of said tube (1) and having a head with multi-axial mobility, **characterized in that** it further comprises an insert (20), capable of sliding into the tube (1), provided, at the proximal end thereof, with a wedge (15) for temporarily immobilizing the orientation of the head of the screw, with said wedge (15) being capable of being introduced through the inside of the tube (1), from the distal end thereof, and having a semi-cylindrical end (8) with a shape matching that of the "U"-shaped notch for receiving a connecting rod provided on the head of the screw.

2. An instrument system according to claim 1, **characterized in that** said immobilizing wedge (15) consists of the proximal end of the insert (20).

3. An instrument system according to claim 1 or claim 2, **characterized in that** said immobilizing wedge (15) has means for connecting with the insert.

4. An instrument system according to the preceding claim, **characterized in that** said insert (20) has at least one guiding rib having a cross-section matching that of at least one guiding groove provided in the cavity formed in said tube (1).

5. An instrument system according to the preceding claim, **characterized in that** said tube consists of two half-shells able to engage with the head (4) of a spinal screw so that at least one of said half-shells (11, 12) can proximally tilt relative to the head of said spinal screw (2).

6. An instrument system according to any one of claims 3 to 5, **characterized in that** said immobilizing wedge (15) has releasable means for fixing on said insert (20).

7. An instrument system according to the preceding claim, **characterized in that** said releasable fixing means consists of a clipping area.

8. An instrument system according to claim 6, **characterized in that** said releasable fixing means consists of a screwing area.

9. An instrument system according to any one of claims 3 to 8, **characterized in that** said insert has an area for screw-locking on said tube (1).

10. An instrument system according to claim 1 or claim 2, **characterized in that** the immobilizing wedge (15) and the insert (20) are one single part.
